# EUROPEAN PATENT APPLICATION

(11) **EP 2 574 666 A1**
(43) Date of publication of application: **03.04.2013**
(21) Application number: 11183972.6
(22) Date of filing: 05.10.2011
(51) Int. Cl.: C12N 5/0781, C12N 5/0783, C12N 5/0784, C07K 16/00, C07K 16/18

(54) **Method for producing antigen-specific antibodies via in vitro immunisation**

(30) Priority: 23.09.2011 EP 11182515
(71) Applicant: Protealmmun GmbH, 13125 Berlin (DE)
(72) Inventor: Bendzko, Peter, Dr., 12623 Berlin (DE); Talke, Anja, Dr., 10555 Berlin (DE); Micheel, Burkhard, Prof.., 17268 Gerswalde (DE)
(74) Representative: Lange, Sven

(57) **Abstract**

The invention relates to an *in vitro* method for production of antigen-specific antibodies, comprising provision of naïve dendritic cells in cell culture, activation of dendritic cells with target antigen, addition of naïve T cells and naïve B cells in co-culture with activated dendritic cells, and activation of T cells and B cells leading to B cell production of said antibodies. The invention therefore relates to an *in vitro* immunization approach by a step-by-step co-cultivation of isolated and activated dendritic cells (DC), helper T cells and B lymphocytes in order to establish an activation process similar to *in vivo* conditions.

## Description

The invention relates to an in vitro method for production of antigen-specific antibodies, comprising provision of naïve dendritic cells in cell culture, activation of dendritic cells with target antigen, addition of naïve T cells and naïve B cells in co-culture with activated dendritic cells, and activation of T cells and B cells leading to B cell production of said antibodies. The invention therefore relates to an in vitro immunization approach by a step-by-step co-cultivation of isolated and activated dendritic cells (DC), helper T cells and B lymphocytes in order to establish an activation process similar to in vivo conditions. The invention also relates to the cooperation of dendritic cells with naïve lymphocyte populations to induce the generation of antigen-specific antibodies in vitro.

The production of monoclonal antibodies by hybridoma technology is dependent on lymphocytes taken from vertebrates which have to be immunized against the corresponding antigen. The invention relates to the replacement of *in vivo* immunization step by an *in vitro* immunization procedure. The invention provides new possibilities for the specific activation of immune cells in order to use them for the generation of antibodies which are of mammalian (preferably human or murine) origin. Bone marrow-derived dendritic cells are loaded with antigen and co-cultured with naïve T and B lymphocytes of non-immunized subjects. The interaction and activation of the different cell types are demonstrated by measuring the expression of specific cell surface markers, the release of activation-dependent interleukins and the secretion of antigen-specific antibodies. According to the method of the present invention dendritic cells process and present antigen fragments, and activate T cells. Activated T cells proliferate and release activation-induced interleukins. B cells maturate under the influence of activated T cells and secrete antigen-specific antibodies.

### Background

Antibodies are not only extremely important components of the immune defence of vertebrates against infectious invaders but are also very important tools in biotechnology and medicine. To induce an effective production of antibodies for an ex *vivo* use it is necessary to know the details going on from the first contact of a vertebrate organism with a foreign antigen to the secretion of antibodies by differentiated B lymphocytes, i.e. plasma cells. Especially if this *in vivo* immunization should be replaced by an *in vitro* procedure the complicated interplay of antigen-presenting cells, T lymphocytes and B lymphocytes in a coordinated tissue culture microenvironment has to be mimicked to allow the necessary cell-cell interactions and interleukin activation. Up to now it is not yet possible to activate a wanted antibody production *in vitro.* A main reason for this failure is the problem that the activation of Th2 cells *in vitro* is not well-established. It is possible to generate Th 1 cell lines (Baker et al., 1979) and antigen-specific T cell lines (Dang 2007) but none of them has a Th2 phenotype which is a key element for the induction of specific B cell responses in order to secrete antigen-specific antibodies.

The experimental procedures so far to induce an artificial immune response *in vitro* are based on the method first described by Mishell and Dutton (1966) which allowed the cultivation of the whole spleen cell population from immunized animals. Later on Borrebaeck (1983) published results on an *in vitro* immunization of non-immune spleen cells and the generation of monoclonal lgM and IgG antibodies. In 1988 a general method for the *in vitro* immunization of purified human B cell populations was described by the same group resulting in the production of human monoclonal antibodies (Borrebaeck et al., 1988). For this approach the cells were stimulated with several cytokines and growth factors and then fused with myeloma cells. This procedure could however be repeated only in a few cases and one has to admit that a general *in vitro* immunization method is not yet feasible. But such an artificial *in vitro* immune response is urgently needed especially to bypass the *in vivo* immunization procedure for the production of monoclonal antibodies.

Since the invention of the hybridoma technique by Köhler and Milstein (1975, 1976) antibodies have received a boost in the treatment and prevention of various diseases and were increasingly used in a broad field of analytical methods as e.g. enzyme-linked immunosorbent assays and immunohistochemistry (Siddiqui 2010). Nevertheless, the production of monoclonal antibodies is an extremely time-consuming and laborious process. The immunization of laboratory animals comprises several weeks of antigen administration, boosting and testing of serum samples. Afterwards the spleen cells have to be fused with myeloma cells followed by a selection process to find the right antibody-producing hybridoma cell. These processes take approximately six months. To combine these requirements in an *in vitro* immunization system would greatly diminish the time frame necessary for the generation of antibody molecules and would also reduce the amount of animals needed.

Human monoclonal antibodies are especially important because they could be valuable potential therapeutics (Wang et al., 2008). Up to now therapeutic monoclonal antibodies of murine origin require "humanization" which is an additional time-consuming and elaborate process (Almagro and Fransson, 2008). Substitution of these processes by an adequate *in vitro* process could permit an acceleration of antibody production and would therefore be a new quality in the generation of antibodies in general.

### Summary of the Invention

In light of the prior art the technical problem underlying the present invention is to provide an in vitro method for production of antigen-specific antibodies that overcomes the disadvantages of those methods known in the prior art.

This problem is solved by the features of the independent claims. Preferred embodiments of the present invention are provided by the dependent claims.

Therefore, the invention relates to an in vitro method for production of antigen-specific antibodies, comprising
- provision of naïve dendritic cells in cell culture,
- activation of dendritic cells with target antigen,
- addition of naïve T cells and naïve B cells in co-culture with activated dendritic cells,
   and
- activation of T cells and B cells leading to B cell production of said antibodies.

In one embodiment the method of the present invention is characterised in that the naive dendritic cells are generated by in vitro differentiation of precursor cells, such as stem cells of multipotent or pluripotent properties, preferably via granulocyte macrophage colony-stimulating factor (GM-CSF) treatment.

In one embodiment the method of the present invention is characterised in that the precursor cells are hematopoietic stem cells or monocytes, preferably obtained from blood, lymph nodes or bone marrow of a subject.

In one embodiment the method of the present invention is characterised in that the target antigen is the antigen against which the antibodies to be produced are directed, so that the antibodies preferably specifically bind the target antigen under appropriate conditions, whereby the target antigen can be any material against which an antibody is directed, such as a protein, peptide, nucleic acid, such as DNA or RNA, sugar molecule, virus and/or bacteria.

In one embodiment the method of the present invention is characterised in that the naïve T cells are naïve helper T lymphocytes, preferably isolated from blood, lymph nodes or spleen of non-immunized subjects.

In one embodiment the method of the present invention is characterised in that the naïve B cells are naïve B lymphocytes, preferably isolated from blood, lymph nodes or spleen of non-immunized subjects.

In one embodiment the method of the present invention is characterised in that
- the dendritic cells are activated via treatment with target antigen, preferably in combination with activation stimuli such as lipopolysaccharide (LPS) or others, to process and present target antigen or fragments thereof on the cell surface,
- the dendritic cells are treated with Th2 inducing stimuli, such as IL-12 siRNA, IL-12 blocking antibody or IL-4, to induce Th2 polarisation of naive T cells
- the T cells are activated via physical contact with the activated dendritic cells, preferably causing proliferation of T cells activated specifically for the target antigen or fragment thereof,
- the B cells are activated by activated T cells to produce antigen-specific antibodies directed against the target antigen or fragment thereof, and/or
- the T and B cells are provided in co-culture either separately, one after the other or simultaneously.

In one embodiment the method of the present invention is characterised in that the B cells, preferably those that produce target antigen-specific antibodies, are fused with myeloma cells to form hybridoma cells.

In one embodiment the method of the present invention is characterised in that the activated antigen-producing hybridoma cells are subsequently selected for mono-clonal cell culture, or that the desired antigen-producing B cells are selected before fusing with myeloma cells.

In one embodiment the method of the present invention is characterised in that
- selection is carried out via a bridge antibody that binds both the hybridoma cell surface and the target antigen, whereby the hybridoma cells are treated with the bridge antibody and target antigen, so that the antigen-specific antibody produced by the hybridoma binds the antigen, and the antigen-specific antibody is detected via binding to a further antibody labelled with fluorescence or via other means; or
- selection is carried out via the target antigen (or desired fragment thereof) labelled with a fluorescent marker or other means, whereby the target antigen is administered to the hybridoma cells and binding between antigen-specific antibody and antigen is measured.

In one embodiment the method of the present invention is characterised in that the antigen-specific antibodies are monoclonal antibodies.

In one embodiment the method of the present invention is characterised in that the method comprises of the following steps, preferably in the following order:
- Collection of blood from subject;
- Isolation of hematopoietic stem cells or monocytes;
- Differentiation of hematopoietic stem cells into naïve dendritic cells in cell culture;
- Activation of dendritic cells with target antigen (antigen of choice);
- Addition of naïve T and B lymphocytes in co-culture;
- Incubation for between 2 and 6 days, preferably between 3 and 5 days, more preferably 4 days;
- Test for target antigen-specific antibody production;
- Fusion of B cells, preferably those that produce target antigen-specific antibodies, with myeloma cells to form hybridoma cells;
- Selection of hybridoma cells that produce target antigen-specific antibodies for monoclonal expansion.

A further aspect of the invention therefore relates to an activated antigen-specific antibody producing B cell, antigen-specific antibody producing hybridoma cell, or cell line derived thereof, that is generated by the method of the present invention.

A further aspect of the invention therefore relates to an antibody produced according to the method of the present invention for use in diagnostic or therapeutic applications.

A further aspect of the invention therefore relates to a kit, comprising components required in order to carry out the method described herein, preferably means for labelling the cells, agents and/or detection antibodies, cell culture medium and/or reagents for driving differentiation of the cells and/or containers or means for cell culture and/or extraction.

The invention therefore relates to a method for producing antigen-specific antibodies, preferably monoclonal antibodies, more preferably human monoclonal antibodies for application in personalised medicine. The method in one embodiment is characterised in that there is no treatment of an animal subject, with the antigen against which the antibodies are directed. The method is preferably an in vitro method.

The invention relates therefore to an in vitro immunisation method, whereby the antigen against which the antibodies are to be directed is provided to a cell culture, in which the immune response proceeds in an analogous manner to an in vivo immune response. The invention relates to an *in vitro* immunization approach by a step-by-step co-cultivation of isolated and activated dendritic cells (DC), helper T cells and B lymphocytes in order to establish an activation process similar to *in vivo* conditions.

In a preferred embodiment, dendritic cells are generated from mammal (preferably mouse or human) bone marrow cells, lymph nodes or peripheral blood lymphocytes and then activated by antigen administration. The activated and antigen-loaded dendritic cells are co-cultured with naïve helper T lymphocytes isolated from blood (of mammals, preferably mouse or human). Naïve B lymphocytes from blood are added to the cultures of DC and T cells to become activated in an antigen-specific manner.

T cell activation resulting from DC-T cell interactions can be shown by CFSE staining, immune fluorescence microscopy and flow cytometric analysis of activation markers. It can be shown that cytokines necessary for interactions between the cultivated immune cells are secreted and that antigen-specific antibodies are produced.

The invention therefore relates to the method as described herein, in addition to the cell lines created via the method as described herein and in the experimental examples section. The invention furthermore relates to the antibodies produced via the method described herein. The invention therefore relates to the use, both in diagnostics and therapy, of the antibodies produced via the method described herein.

### Detailed description of the invention

The method of the present invention relates to the in vitro production of antigen-specific antibodies from mammalian cell culture systems. The invention preferably relates to generating antibodies from human cell culture. The method allows generation of antibodies from organisms that only rarely may be used to generate antibodies via hyperimmunization against special antigens. A personalised medicine approach can be achieved with the present invention, whereby therapeutic antibodies are produced in vitro (in cell culture) in cells derived originally from the patient. The advantage is that the risk of an adverse immune reaction of the patient after treatment is significantly reduced, even in comparison to humanised (non-personalised) antibodies.

In one embodiment the method utilises blood collection from patients. As an alternative source bone marrow or cord blood could be used. Hematopoietic stem cells are subsequently selected, or for example monocytes could be used, before differentiation of the cells into dendritic cells. The differentiation can be achieved via various treatments of the cells; a preferred embodiment is described below. The dendritic cells are subsequently activated with the antigen of choice, against which the anti-bodies are to be directed. Naïve T- and B- cells are added, either together before or after the addition of each other or the antigen of interest, preferably after antigen treatment. The cells are then incubated under appropriate conditions for 2 to 6 days, preferably 3 to 5 days, more preferably 4 days. Antibody production is then assayed.

In one embodiment of the invention, the antigen producing cells must then be selected, in order to select those cells that produce the desired antibodies. Various methods are known in the art that could be applied in order to select those cells, and thus generate clones of those cells that produce the desired antibody. Fusion of the antibody producing B cells can be achieved in order to immortalise the cells. Such fusion approaches are known in the art and can be applied in this case.

The terms "naïve" and "immature" cells, such as dendritic, B and/or T cells, have been used interchangeably in the present application.

### Examples

The following examples represent a preferred method of the invention but provide no limitation to the scope of the invention. The examples as carried out here in mouse model organisms relate to one embodiment of the invention, although the invention can also be carried out with cells of human origin, as is desired, in order to provide humanised antibodies for therapy and/or diagnostic uses.

### Materials and methods used in the examples provided herein:

### Animals

Mice of the age of 8 to 12 weeks were used for the experiments. Balb/c mice were bred in our facility at Potsdam University (Potsdam, Germany). Transgenic mice with a Balb/c background expressing a T cell receptor specific for ovalbumin (DO 11.10) were kindly provided by R. Baumgrass (DRFZ, Berlin, Germany).

### Preparation of murine dendritic cells by differentiation from hematopoietic stem cells

Bone marrow cells from femurs and tibiae of Balb/c mice were isolated as previously described (Lutz et al., 1999). The cells were washed in 20 ml RPMI 1640 (Biochrom AG, Berlin, Germany) and resuspended in 24 mL RPMI 1640 complete medium supplemented with 2 mM L-glutamine (Biochrom AG, Berlin, Germany), 50 pM 2-mercaptoethanol (Roth, Karlsruhe, Germany), 10 % heat-inactivated fetal calf serum (Biochrom AG, Berlin, Germany) and 20 ng/mL recombinant mouse GM-CSF (Strathmann Biotec AG, Hamburg, Germany).

In further experiments the culture supernatant of a cell line transfected with the gene for murine GM-CSF was used instead of recombinant mouse GM-CSF. Adding 5% culture supernatant containing GM-CSF generated the same quantity as did 20 ng/mL recombinant mouse GM-CSF (Lutz et al., 1999). The cells (1x10⁶ cells per well in 2 mL complete medium) were incubated in 6-well plates (TPP, Trasadingen, Switzerland) for seven days at 37°C and 5 % CO₂. On day two after the preparation 2 mL fresh complete medium with 5 % GM-CSF containing supernatant were added to the wells. On day four 2 mL of culture supernatant per well were removed and centrifuged for 10 minutes at 200x g. Then the cell pellet was resuspended in 24 mL of fresh complete medium with 5 % GM-CSF containing supernatant and 2 mL per well were transferred back to the 6-well plates. The differentiation of hematopoietic stem cells into immature dendritic cells was completed at day seven.

### Isolation and enrichment of naïve lymphocyte populations

Naïve T and B cells were isolated from the spleen of Balb/c and DO 11.10 mice, when indicated. Isolation and enrichment of naïve lymphocyte populations were performed by using the CD4⁺-CD62L⁺-T cell Isolation Kit II and the B cell Isolation Kit purchased from Miltenyi Biotec (Bergisch-Gladbach, Germany). The procedure was carried out according to the instructions of the producer using a magnetic cell sorting device (MACS) from the same company.

### Cultivation and stimulation of immune cells for in vitro immunization

Naïve lymphocytes (3x10⁵ T cells and 3x10⁶ B cells per well) were added to the immature dendritic cells. Then cultures were stimulated in different ways, i.e. addition of either 11.25 µg/mL ovalbumin (Sigma, Saint Louis, USA), 1 µg/mL lipopolysaccharide (LPS, Sigma, Saint Louis, USA), both ovalbumin and LPS or without stimulation. After that the cells were cocultivated as before at 37°C and 5 % CO₂ for several days. Culture supernatants (500 µL) were collected every day and used for the determination of interleukins and ovalbumin specific antibodies.

### Activation of immature dendritic cells

For analysing antigen processing immature dendritic cells were generated as described above. At day seven the immature dendritic cells were challenged with various concentrations of DQ-ovalbumin (Invitrogen, Karlsruhe, Germany) ranging from 0.5 to 10 µg/mL. Control cultures were left untreated. Furthermore CHO cells (1x10⁶ cells per well) were either challenged with 5 µg/mL DQ-ovalbumin or left untreated to serve as an additional control. Cells were analysed by flow cytometry using the FACSCalibur (BD Biosciences, Heidelberg, Germany) at different time points after challenge.

To monitor the secretion of IL-12 as a marker for activation dendritic cells were isolated and generated as described above. Culture supernatants were collected at different time points after generation. At day eight dendritic cells were stimulated with 11.25 µg/mL ovalbumin, 1 µg/mL LPS, both ovalbumin and LPS or left untreated. Interleukin 12 in the culture supernatants was detected using the Mouse Th1/Th2 9-Plex Tissue Culture Kit (Meso Scale Discovery, Gaithersburg, USA).

For analysing the expression of activation markers CD80 and CD86 immature dendritic cells were generated as described above. At day seven the immature dendritic cells were challenged with either 11.25 µg/mL ovalbumin (Sigma), 1 µg/mL lipopolysaccharide LPS (Roth, Karlsruhe, Germany), both ovalbumin and LPS or left untreated. Cells were harvested 24 hours after stimulation and centrifuged for five minutes at 200x g. Supernatants were discarded and cells were resuspended in 100 µL staining buffer containing 2 µg/mL APC-labelled hamster-anti-mouse-CD80 antibody (eBioscience, San Diego, USA). After incubation for 15 minutes at 4°C in the dark cells were centrifuged for five minutes at 200x g and then resuspended in 100 µL staining buffer containing 2.5 µg/mL FITC-labelled rat-anti-mouse-CD86 antibody (Abcam Inc., Cambridge, USA). Cells were incubated at 4°C in the dark, centrifuged for five minutes at 200x g and resuspended in 300 µL staining buffer.

### Fluorescence microscopy

For immunofluorescence microscopy cover slips were sterilized with ethanol (100 %), applied to 6-well plates and coated with 50 µg/mL poly-l-lysin (Sigma) in PBS for one hour at room temperature. The slips were washed with 1 mL PBS per well and dried at room temperature overnight under sterile conditions. Immature dendritic cells and naïve T cells were cultured on these cover slips in the same concentrations as described for morphological investigations. The cells were incubated for one hour at 37°C. After that the plate was centrifuged at 200x g for five minutes, medium was removed and the cells were fixed in 4% paraformaldehyde (1 mL/well, pH 7.4, Sigma) for 15 minutes at room temperature. The cover slips were washed two times with 1 mL PBS per well. To avoid unspecific bindings during the staining procedure the cover slips were incubated in 2 mL/well PBS/1% BSA for 20 minutes at room temperature. Staining of the actin cytoskeleton was performed by incubating the cells with 100 µL Bodipy-Phalloidin (Molecular Probes, Oregon, USA) diluted 1:40 in PBS/1 % BSA for 30 minutes at 37°C. After washing two times with 1 mL PBS/ well the cover slips were incubated with 0.5 pg biotinylated rat-anti-mouse-CD90.2 antibody (eBioscience, San Diego, USA) diluted in 100 µL PBS/1% BSA for 30 minutes at 37°C. Cover slips were washed again two times with 1 mL PBS/ well and then incubated with 0.2 µg Pacific Blue labelled streptavidin (Molecular Probes, Eugene, USA ) in 100 µL PBS/1 % BSA for 30 minutes at 37°C. The slips were washed two times with 1 mL PBS/well and 2 mL distilled water and then mounted in Mowiol 4.88 (Polysciences Europe GmbH, Eppelheim, Germany).

### Flow cytometry analysis of T cell proliferation

For analysing T cell proliferation naïve T cells from D011.10 mice were isolated as described above and labelled with carboxyfluorescein succinimidyl ester (CFSE, Sigma-Aldrich, Germany). In brief, T cells were washed with RPMI medium twice. After that naïve T cells were resuspended in 1 mL 5µM CFSE solution and incubated for 15 minutes at room temperature in the dark. The CFSE-labelled T cells were added to immature dendritic cells (3x10⁵ T cells/well) and cultures were challenged with either 11.25 µg/mL ovalbumin, 1 µg/mL LPS, both ovalbumin and LPS or left untreated. Cells were cocultivated for seven days at 37°C and 5% CO2 and were harvested every day for flow cytometry analysis.

### Flow cytometry analysis of B cell activation

Activation status of B cells cocultured with dendritic cells and T cells was determined by flow cytometry analysis of various activation markers. Therefore, cocultures of dendritic cells, T cells and B cells were stimulated with 5 µg/mL ovalbumin and cells were harvested at different time points for flow cytometry. Briefly, dendritic cells, T cells from D011.10 mice and B cells were generated and isolated as described above. Dendritic cells and B cells were stimulated with 5 µg/mL ovalbumin for two hours at 37°C, 5 % CO₂, washed with RPMI 1640 two times and then resuspended in complete culture medium. All three cell types were cocultivated for at least four additional days. During cultivation period, culture supernatants were collected and cells were harvested for flow cytometric analysis. Cells were stained with either 2.5 µg/mL FITC-conjugated rat-anti-mouse-CD19-antibody (eBioscience, San Diego, USA) or APC-conjugated rat-anti-mouse-CD19-antibody (AbD Serotec, Duesseldorf, Germany) in 100 µL staining buffer for 15 minutes at 4°C in the dark. Cells were centrifuged at 200x g for five minutes and stained with either 1 µg/mL FITC-conjugated rat-anti-mouse-CD38-antibody (AbD Serotec, Duesseldorf, Germany) or 2.5 µg/mL rat-anti-mouse-IgD-antibody (eBioscience, San Diego, USA) in 100 µL staining buffer for 15 minutes at 4°C in the dark, respectively. Cells were then centrifuged at 200x g for five minutes and resuspended in 300 µL staining buffer.

### Determination of interleukins in the culture supernatants

For the determination of interleukins in the culture supernatants the
Mouse Th1/Th2 9-Plex Tissue Culture Kit (Meso Scale Discovery, Gaithersburg, USA) was used. This kit allows simultaneous detection of mouse IL-1 beta, IL-2, IL-4, IL-5, IL-10, IL-12 (total), IFN-gamma, TNF-alpha and mouse KC (CXCL1). The detection is based on electrochemiluminescence. The assay was performed according to the manufacturers' instructions. Briefly, culture supernatants and calibration mix were transferred onto pre-coated 96-well plates (25 µL /well) and incubated with vigorous shaking for one hour at room temperature. Detection antibody mix was added (25 µL/well) and incubated with vigorous shaking for one hour at room temperature. Following that the plates were washed three times with PBS/0.05% Tween20 (300 µL/well) and reading buffer was added (150 µL/well). The plates were analysed immediately using the Sector Imager 6000 (Meso Scale Discovery, Gaithersburg, USA). All experiments were performed in duplicates. Mean values and standard deviation were calculated.

### Detection of antigen-specific antibodies in culture supernatants

To detect antibodies with bound antigen (due to the ovalbumin added during the stimulation phase) in the collected culture supernatants of stimulated and unstimulated cultures an enzyme immunoassay according to the following incubation sequence was performed. Therefore, 96-well plates (Greiner,Bio-One GmbH, Solingen, Germany) were coated with 50 µL/well of 5 µg/mL goat-anti-mouse-Ig-antibody (Dianova, Hamburg, Germany) at 4°C overnight. Wells were blocked with 100 pL/ well of PBS containing 5% NCS for 120 min at room temperature. Diluted culture supernatants (possibly containing antibodies with bound ovalbumin) were added for 60 min at room temperature. For the detection of antibody-ovalbumin complexes the wells were incubated with 50 µl of HRP-conjugated polyclonal rabbit-anti-ovalbumin-antibody (Seramun Diagnostica GmbH, Heidesee, Germany) for 60 min at room temperature. Following that, 50 µl / well substrate solution containing 3,3'-5,5'-tetramethylbenzidine was added for 5 min and the reaction was stopped by adding 50 µl/ well 1 M sulphuric acid. Absorbance at 450 nm was measured by a Paradigm plate reader (Beckman&Coulter, Krefeld, Germany).

### Statistical Analysis

Statistic evaluation of the experimental data was done with a t-test calculator software from GraphPad Inc. (San Diego, CA, USA) as well as with the t-test function of Microsoft Office Excel 2007.

### Experimental Results:

### Activation of dendritic cells in vitro

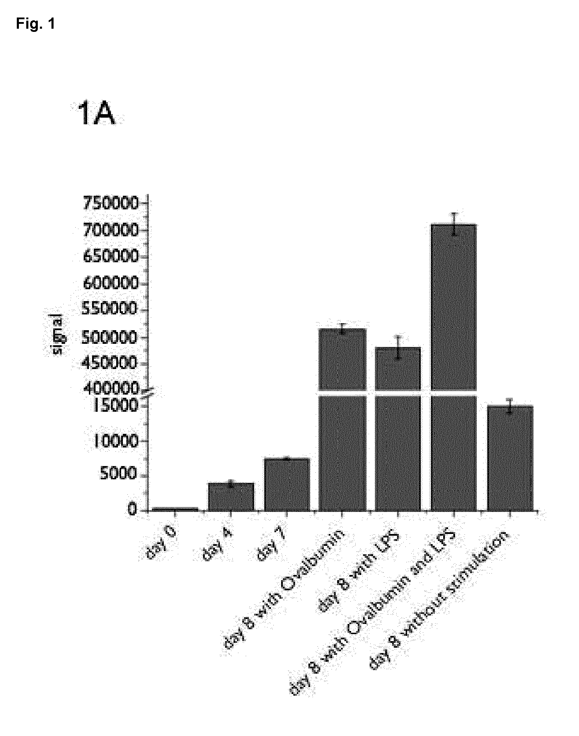

First we investigated the secretion of IL-12 by dendritic cells since IL-12 is one of the major interleukins produced upon activation. To examine whether bone marrow derived dendritic cells are activated by antigen stimulation the cells were challenged with different stimuli, i. e. ovalbumin, LPS, both ovalbumin and LPS or left untreated. We found that both, stimulation with ovalbumin and stimulation with LPS lead to activation of the dendritic cells resulting in a high secretion of IL-12. Highest signals were detected when dendritic cells were stimulated with ovalbumin and LPS simultaneously (**Fig. 1A**). n=2, ***, p < 0.001; **, p < 0.01 (unpaired Student's t test)

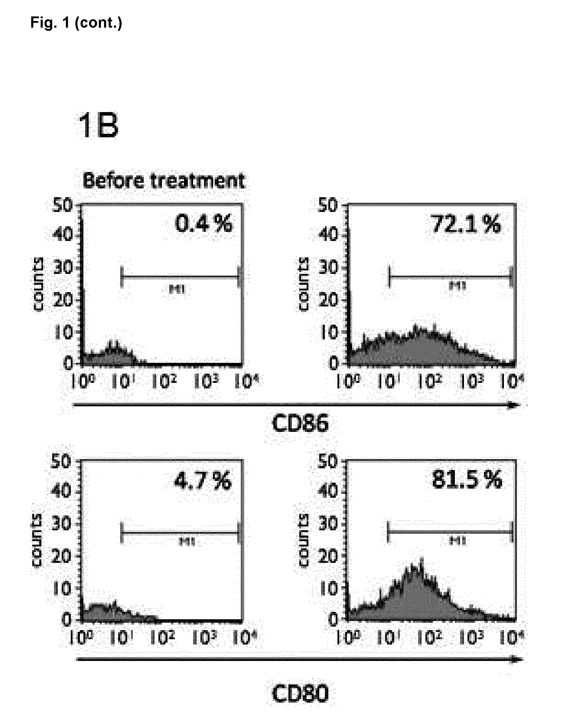

Furthermore we analyzed the expression of surface activation markers as CD80 and CD86. Flow cytometry analysis revealed that both activation markers, CD80 and CD86, are up-regulated after stimulation of the cells. Briefly, 0.4 % of the cells expressed CD86 before stimulation, whereas 72.1 % of the cells expressed CD86 after being stimulated. The number of CD80-expressing cells increased from nearly 5 % before treatment to more than 81 % after treatment (**Fig. 1B**). The data presented are representatives out of three independent experiments.

### Processing and presentation of DQ-ovalbumin by isolated dendritic cells

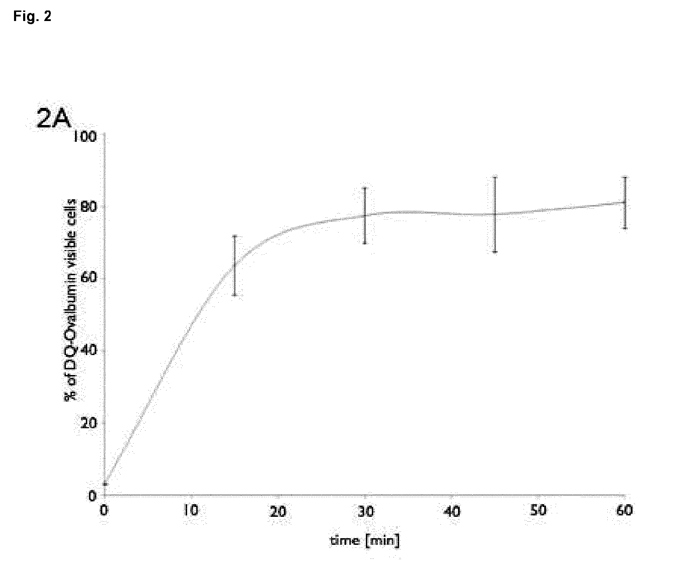

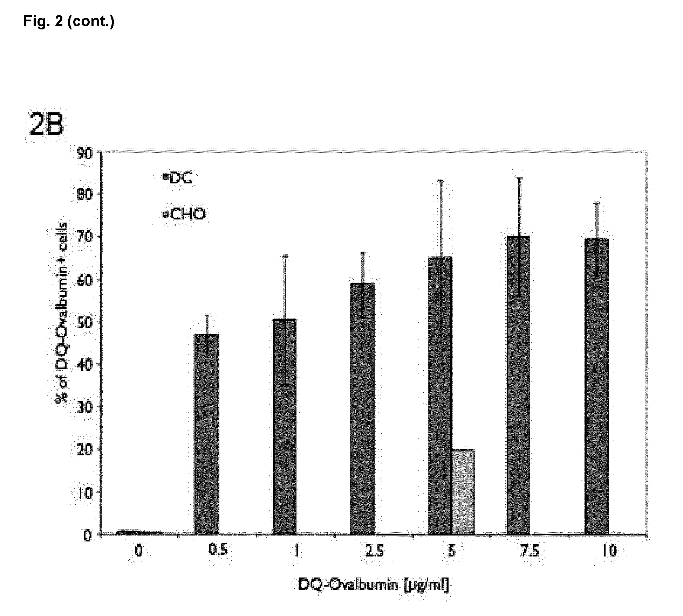

In order to create dendritic cells that can be used for an *in vitro* immunization, we investigated the ability of generated dendritic cells to process and present internalized antigen. For this purpose the cells were stimulated with DQ-ovalbumin. DQ-ovalbumin is a self-quenching ovalbumin conjugate that exhibits fluorescence upon proteolytic digestion. As shown in **Fig. 2A** nearly 60% of the viable dendritic cells had processed DQ-ovalbumin after 15 minutes of stimulation. Thirty minutes after application of DQ-ovalbumin, 82% of the dendritic cells had processed and presented the antigen. Following that, we investigated the amount of antigen necessary to activate a maximal number of dendritic cells. Therefore dendritic cells and CHO cells were incubated with various concentrations of DQ-ovalbumin ranging from 0.5 up to 10 µg/mL. As shown in **Fig. 2B** almost 80% of the dendritic cells have taken up and presented the antigen at a concentration of 5 µg/mL DQ-ovalbumin. Using higher concentrations did not increase the number of activated dendritic cells that present the antigen. The data were summarized out of two independent experiments and presented as mean ± SEM.

Summing up these results, we could show that the *in vitro* generated and activated dendritic cells are able to fulfil their functions as antigen presenting cells needed in an immune reaction.

### Activation of naïve T helper cells by activated dendritic cells

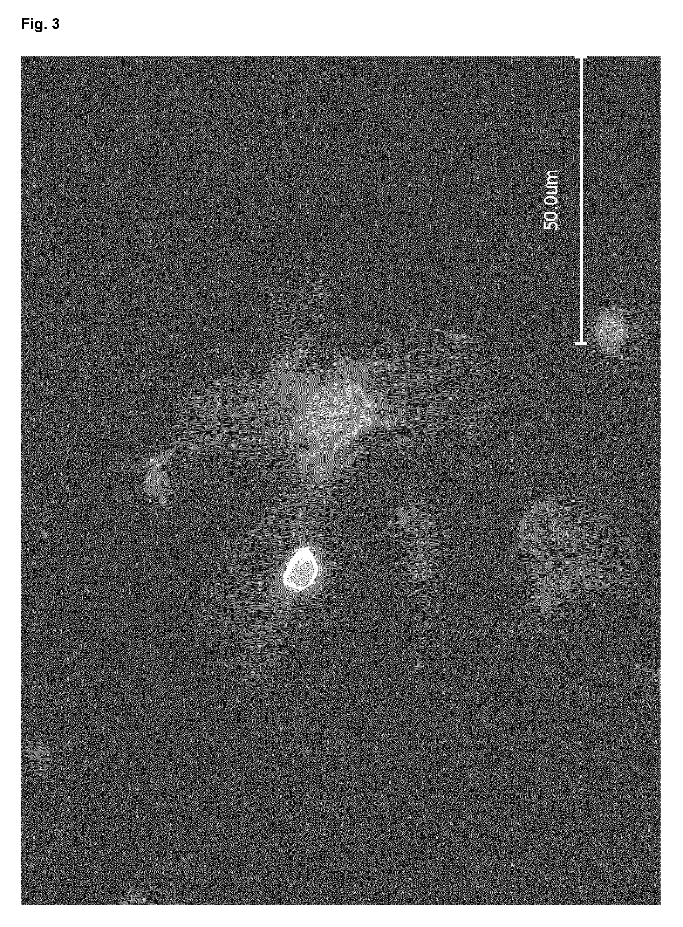

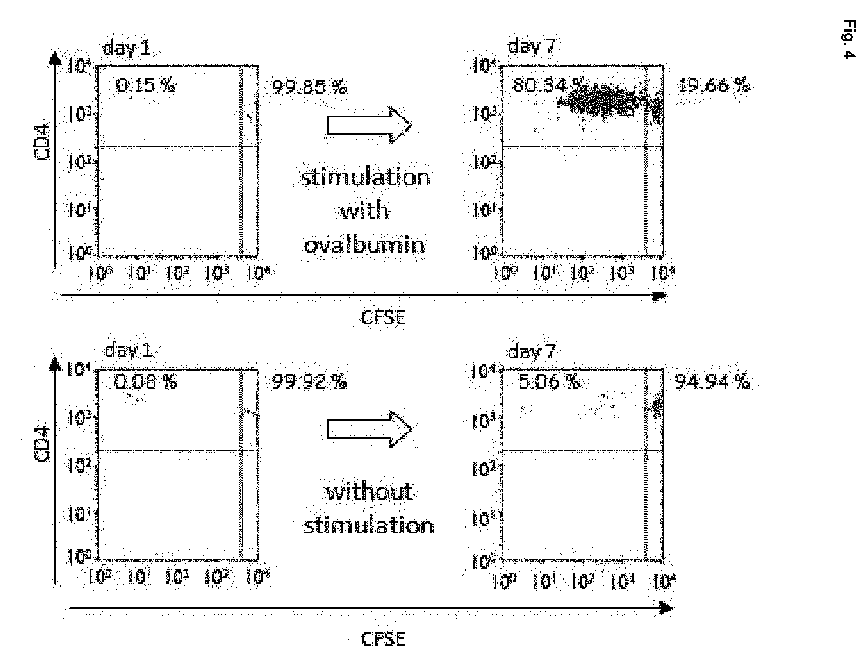

For the experimental outline activated dendritic cells and naïve T helper cells were cocultured in order to induce the necessary cell-cell contacts for activation. The cell-cell contacts were visualized by fluorescence microscopy (**Fig. 3**). By cocultivation of both cell types we could show the specific interaction between antigen-presenting cells and T helper cells which is an essential step in the immune cascade *in vivo.* Following that we examined whether the T helper cell population proliferates after the cell-cell contacts. For this T helper cells were labeled with CFSE and flow cytometry was performed at different time points. At day seven of cocultivation we could detect strong T helper cell proliferation in cultures stimulated with ovalbumin as antigen or with ovalbumin and LPS added simultaneously (**Fig. 4**). More than 80% of the T helper cells in cultures stimulated with ovalbumin proliferated upon stimulation, whereas in unstimulated cultures only 5% of the T cells proliferated. In cultures stimulated with ovalbumin and LPS simultaneously 30-50% of the T helper cells showed proliferation upon stimulation. The data presented are representatives out of four independent experiments.

These results show that *in vitro* generated and activated dendritic cells are able to induce T helper cell activation *in vitro.*

### Activation of B lymphocytes by activated T helper cells

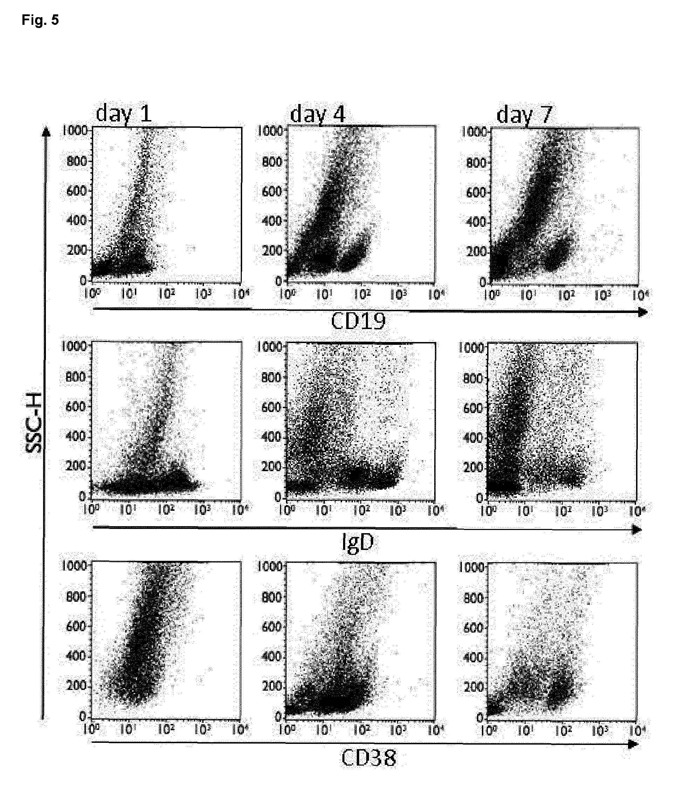

To investigate whether B lymphocytes were attracted by activated T helper cells *in vitro* cocultivated B cells were examined via flow cytometry for the expression of the surface markers CD19, CD38 and IgD. The different expression patterns were monitored over seven days of cultivation. Already at day three we could detect two different B cell populations which expressed CD19, CD38 and IgD in moderate or high concentrations. Later on these subpopulations showed a drift to the higher expression (**Fig. 5**) which indicates a partial activation of B lymphocytes in *vitro.* The data presented are representatives out of four independent experiments.

### Detection of interleukins in the supernatants of cultivated immune cells

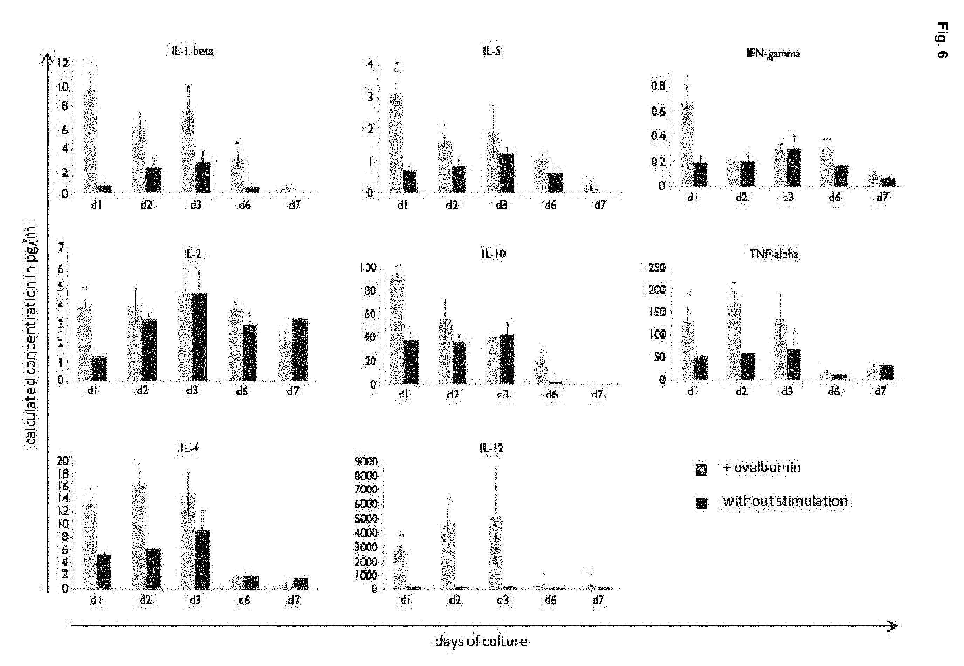

To verify whether the partial activation of B lymphocytes in vitro was due to the establishment of a Th2 immune response in culture the cytokine patterns were analysed. Cultures of dendritic cells, naïve T cells and naïve B cells were, therefore, either stimulated with ovalbumin or left untreated and were analysed especially for the synthesis of the cytokines IL-4 and IL-5 at different time points of cultivation. In culture supernatants of stimulated cultures we could detect significantly higher amounts of IL-4, IL-5 and IL-10 which indicate the activation of Th2 cells in our *in vitro* cultures (**Fig. 6**). Additionally we could detect high amounts of IL-12 in stimulated cultures as a consequence of the activation of dendritic cells. Proinflammatory cytokines as IFNy, TNFa and IL-1 b showed significant differences at the first day of cultivation. The data presented are representatives out of three independent experiments. ***, p < 0.001, **, p < 0.01, *, p < 0.05 (unpaired Student's t test)

### Detection of antigen-specific antibodies in the culture supernatants

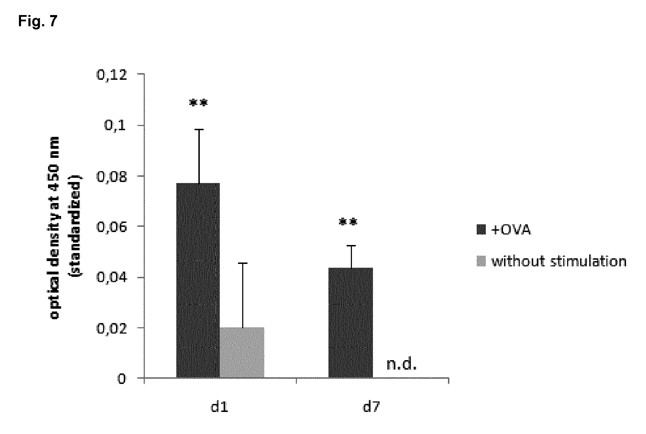

The detection of antigen-specific antibodies was performed by an enzyme immunoassay which detected ovalbumin-antibody complexes. In stimulated cultures we observed a significantly higher amount of anti-ovalbumin-antibodies which had bound ovalbumin compared to unstimulated cultures (**Fig. 7**). These data showed a successful activation of naïve B cells to produce antibodies *in vitro* induced by interactions of activated dendritic cells and T lymphocytes. n=5, **, p < 0.006 (unpaired Student's t test)

### Discussion

To transfer an immune reaction to *in vitro* systems requires the activation of all involved cell populations in a similar way as under natural in vivo conditions. In contrast to previous in *vitro* immunization approaches we tried to activate the different cell types in a coordinated step-by-step manner and verified the activation status and cell-cell interactions by determining the expression of cell surface markers and the release of interleukins and antibodies. We generated naïve dendritic cells from hematopoietic stem cells and loaded them with ovalbumin as antigen. Dendritic cells are the most potent antigen-presenting cells and are strongly activated by glycosylated or mannosylated antigens. Ovalbumin has one mannosylated side chain and seems, therefore, to be an appropriate model antigen for an *in vitro* immunization approach (Burgdorf et al., 2006). The optimum antigen presenting capacity of *in vitro* generated dendritic cells was shown after 30 minutes with an antigen concentration of 5 pg/mL. In our experiments we observed a saturation curve in antigen uptake and processing which resembles the *in vivo* behaviour (Germain and Jenkins, 2004). Together with an upregulation of activation markers and the secretion of IL-12 we could show that the generated dendritic cells were capable to present antigen and initiate a T cell response *in vitro.* The interaction between mature dendritic cells and naïve T cells was directly visualized by immunofluorescence staining. The activation of T cells could be shown by a clear-cut proliferation of this cell population.

It is assumed that different hierarchies in antigen presentation, signalling and receptor engagement play an important role in the induction of Th2 mediated immune responses which are characterized by a specific cytokine profile as e.g. IL-4, IL-5 and IL-13 (Pulendran et al., 2010). In the supernatants of our *in vitro* approaches we were able to detect significantly higher IL-4 and IL-5 concentrations compared to unstimulated cultures which could indicate a Th2 profile. Since naïve T cells are not able to synthesize IL-4 before they are activated we assume that the source of this cytokine in *our in vitro* experiments was an activated Th2 population. These presumable Th2 cells were obviously able to activate naïve B lymphocytes. After addition of naïve B lymphocytes to the cultures of antigen-loaded and activated dendritic cells and activated T cells (presumably containing Th2 cells) an increased expression of CD19, CD38 and membrane-bound IgD could be shown. Actually, two expression patterns - moderate and high -were observed in the B cell population which indicates an activation of at least some parts of this population. CD19 is a coreceptor on B cells which is important for ligation with the B cell receptor after antigen recognition. This ligation induces the humoral immune response and mice lacking the receptor genes showed an impaired B cell immunity to T-dependent and T-independent antigens (Barrington et al., 2009). The functional significance of CD38 expression on normal B lymphocytes is not well understood but there is evidence for different expression patterns during B cell maturation. A low expression of CD38 was shown for mature naïve B lymphocytes whereas the activation of these B lymphocytes induced a re-expression of CD38 into a high-expressing population. These cells were differentiated into germinal centre B cells (Liu et al., 1997). IgD expression seems to be also important for mature B cells and is induced by IL-4 and IL-10 (Levan-Petit et al., 1999). There is evidence that IgD has a regulatory function in B cell homeostasis, modulates B cell selection and fine-tunes humoral responses (Geisberger et al., 2006). All three markers can therefore be taken in our experiments as indicators for an *in vitro* activation and differentiation of B cells. This activation is underlined by the fact that we found ovalbumin-specific antibodies.

Antigenic peptides can be linked to lysine residues by using MAP systems as described by Fujiki et al. (2010) in order to induce higher antibody responses *in vitro.* Furthermore, a more successful Th2 differentiation can be obtained by a specific activation with Th2 antigens such as e.g. SEA *(Schistosoma* egg antigen) and by an inhibition of the Th1 differentiation e.g. by downregulating the IL-12 secretion of dendritic cells by means of specific siRNA. Additional experiments in progress show that different supporting media and structures (mesenchymal stem cells, growth factors, biomimetic support layers which mimic the three-dimensional arrangement of the cells in lymph nodes etc.) may help to initiate a more successful *in vitro* immune response. To realize a continuous *in vitro* immunization approach several additional improvements can be made to the method described herein, such as i.e. enhancing the short lifetime of B and T lymphocytes and improving the constant supply of new cell populations which can be activated against specific antigen. There are hitherto no known solutions to differentiate *in vitro* a pluripotent hematopoietic stem cell into a B or T lymphocyte expressing specific immune receptors at the cell surface. It is not yet known how to activate stem cells to differentiate into a lymphocyte *in vitro* and how to start somatic recombination. But since there are some B cell lines e.g. the human line BI 2 (Poltoratsky et al., 2007) and the chicken line DT40 (Magari et al., 2010) which hypermutate their lg genes *in vitro* it should be possible to include some of the molecular mechanisms of these cell lines in an *in vitro* immunization approach to produce monoclonal antibodies.

In summary, the invention relates to an orchestrated step-by-step activation of dendritic cells, T cells and B cells to induce production of antigen-specific antibodies *in vitro.* This approach could be very helpful to shorten the time frame of conventional immunization procedures from approximately three months to a few days and further to accelerate the generation of antibody molecules.

### Description of the Figures:

**Figure 1****: (A) Secretion of IL-12 following activation**. Dendritic cells were generated from bone marrow cells and challenged with various stimuli at day eight, i.e. 11.25 µg/mL ovalbumin, 1 µg/mL LPS, in combination or left untreated. Culture supernatants were collected during generation and stimulation, and secretion of IL-12 was measured in duplicates via MSD multiplex platform. ***, p < 0.001; **, p < 0.01 (unpaired Student's *t* test). **(B) Expression of CD80 and CD86 by dendritic cells as markers of activation**. Dendritic cells were generated and at day seven challenged with either 11.25 µg/mL ovalbumin, 1 µg/mL LPS, both ovalbumin and LPS or left untreated. Cells were harvested 24 hours after stimulation and stained with 2 µg/mL APC-conjugated hamster-anti-mouse-CD80-antibody or 5 µg/mL FITC-conjugated rat-anti-mouse-CD86-antibody for 15 minutes at 4°C in the dark. Cells were then centrifuged for five minutes at 200x g, resuspended in 300 µL staining buffer and analysed via flow cytometry. The data presented are representatives out of three independent experiments.
**Figure 2****: (A) Antigen processing and presentation by activated dendritic cells ― time response.** Dendritic cells were generated and stimulated with 5 µg/mL DQ-ovalbumin. Cells were incubated at 37°C and 5% CO₂, and harvested at 15, 30, 45 and 60 minutes after stimulation. For flow cytometry analysis the cells were centrifuged for five minutes at 200x g and then resuspended in 300 µL staining buffer. **(B) Antigen processing and presentation by activated dendritic cells ― concentration dependence.** At day seven immature dendritic cells were challenged with various concentrations ranging from 0.5 to 10 µg/mL DQ-ovalbumin or left untreated. CHO cells (1x106 cells per well) were either challenged with 5 µg/mL DQ-ovalbumin or left untreated to serve as a control. Cells were analysed in duplicates via flow cytometry. The data were summarized out of two independent experiments and presented as means ± SEM.
**Figure 3****: lmmunfluorescence staining of activated dendritic cells and T cells.** Immature dendritic cells (1x10⁶ cells/well) were activated with ovalbumin. Naïve T cells (3x10⁵ cells/well) were added. Cells were cultured on poly-I-lysin (50 µg/mL) coated cover slips and fixed with 4% paraformaldehyde (15 min, RT). The slides were saturated with PBS/1% BSA (20 min, RT) and stained with 100 µL Bodipy-Phalloidin (1:40 diluted in PBS/1% BSA, 30 min, 37°C) and biotin-conjugated rat-anti-mouse CD90.2-antibody and Pacific Blue-conjugated streptavidin (5 µg/mL in PBS/1% BSA, 30 min, 37°C). Staining was analysed by fluorescence microscopy using the BZ 8000 K microscope (Keyence, Neu-Isenburg, Germany) at a magnification of 60x and 60x optical zoom (bar 50 µm).
**Figure 4****: T cell proliferation after activation by activated dendritic cells**. Naïve T cells from transgenic D011.10 mice were isolated, labelled with 5 µM CFSE and then added to cultures of immature dendritic cells (3x10⁵ T cells/well). Cultures were stimulated with 11.25 µg/mL ovalbumin, 11.25 µg/mL ovalbumin and 1 µg/mL LPS or left untreated. Cells were cultivated for seven days and CFSE fluorescence was monitored by flow cytometry. The data presented are representatives out of four experiments.
**Figure 5****: B cell activation by activated T cells**. Naïve T cells from transgenic D011.10 mice and naïve B cells from Balb/c mice were isolated and added to cultures of immature dendritic cells. Cultures were either stimulated with 5 µg/mL ovalbumin or left untreated. Cells were harvested daily to perform flow cytometric analysis of cell surface markers CD19, CD38 and membrane-bound IgD. Cells were stained with 2.5 µg/mL FITC-conjugated rat-anti-mouse-CD19-antibody (eBioscience, San Diego, USA), 2.5 µg/mL APC-conjugated rat-anti-mouse-CD19-antibody (AbD Serotec, Duesseldorf, Germany), 1 µg/mL FITC-conjugated rat-anti-mouse-CD38-antibody (AbD Serotec, Duesseldorf, Germany) or 2.5 µg/mL rat-anti-mouse-IgD-antibody (eBioscience, San Diego, USA) in 100 µL staining buffer and were incubated for 15 minutes at 4°C in the dark. The data presented are representatives out of four experiments.
**Figure 6****: Detection of interleukins in culture supernatants of activated dendritic cells, T cells and B cells**. Dendritic cells, B and T lymphocytes were cultivated for seven days and culture supernatants were collected each day. Cytokine analysis was performed using the Mouse Th1/Th2 9-Plex Tissue Culture Kit (Meso Scale Discovery, Gaithersburg, USA). The data presented are representatives out of three experiments and presented as means ± SEM. ***, p < 0.001, **,p< 0.01, *,p< 0.05
**Figure 7****: Detection of antigen-specific antibodies in culture supernatants of activated B cells.** Dendritic cells, B and T lymphocytes were cultivated for seven days and culture supernatants were collected each day. 96-well plates were coated with 5 µg/mL goat-anti-mouse-Ig-antibody (Dianova, Hamburg, Germany) at 4°C overnight. After blocking with PBS/ 5% NCS for 120 min at room temperature culture supernatants were diluted with blocking buffer and were incubated for 60 min at room temperature. For detection wells were incubated with 50 µl HRP-conjugated polyclonal rabbit-anti-ovalbumin-antibody (Seramun Diagnostica GmbH, Heidesee, Germany) for 60 min at room temperature. Substrate solution containing 3,3'-5,5'-tetramethylbenzidine was used for signal generation. Plates were analyzed with Paradigm plate reader (Beckman&Coulter, Krefeld, Germany). The data presented are representatives of five experiments and are given as the mean ± S.E.M. **, p < 0.006 (unpaired Student's *t* test)

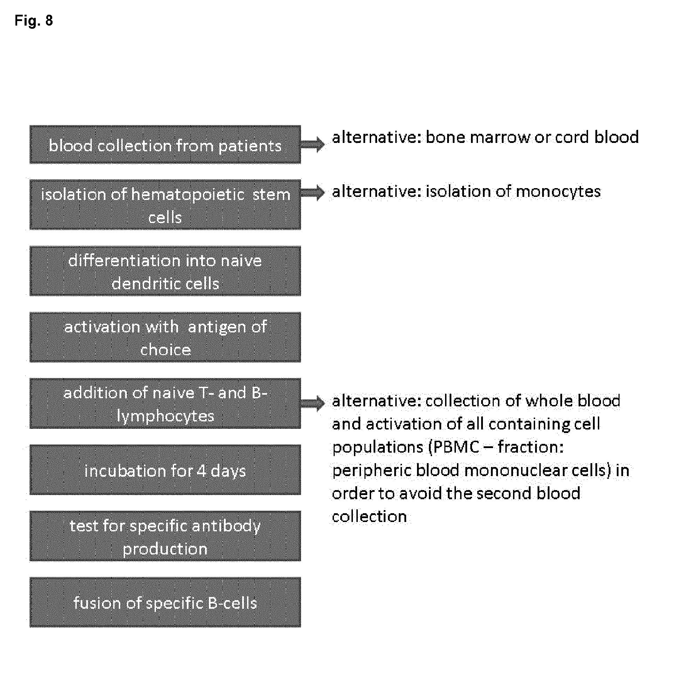

**Figure 8****: Flow chart of method of the present invention.** The steps provided in Fig. 8 represent a preferred embodiment of the invention and can be carried out either in part, for example without necessarily containing all steps shown, or in a different order as demonstrated in Fig 8 if required.

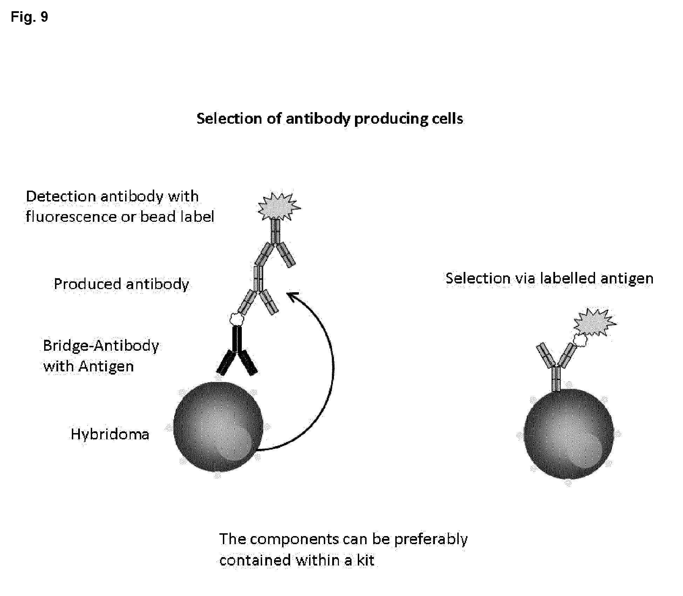

**Figure 9****: Selection of antibody producing cells.** Two possible approaches for selecting the desired cells are shown in Figure 9. In a preferred embodiment a bridge antibody with antigen is applied, whereby the bridge antibody recognises the hybridoma cell. The produced antibody then binds the antigen; which can be detected with a detection antibody labelled with fluorescence or via other means. Otherwise, selection of the hybridoma can be carried out using an antigen labelled with a fluorescent marker or other means.

### References:

Almagro, J. C. and Fransson, J., 2008. Humanization of antibodies. Front. Biosci. 13, 1619-1633.
Baker, P.E., Gillis, S. and Smith, K.A., 1979. Monoclonal cytolytic T-cell lines. J. Exp. Med. 149, 273-278.
Barrington, R. A., Schneider, T. J., Pitcher, L. A., Mempel, T. R., Ma, M., Barteneva, N. S., Caroll, M. C., 2009. Uncoupling CD21 and CD19 of the B cell coreceptor. Proc. Natl. Acad. Sci. 106, 14490-14495.
Borrebaeck, C. A., 1983. In vitro immunization for the production of antigen-specific lymphocyte hybridomas. Scand. J. Immunol. 18, 9-12.
Borrebaeck, C. A., Danielsson, L. and Möller, S. A., 1988. Human monoclonal antibodies produced by primary in vitro immunization of peripheral blood lymphocytes. Proc. Natl. Acad. Sci. 83, 3995-3999.
Burgdorf, S., Lukacs-Kornek, V. and Kurts, C., 2006. The mannose receptor mediates uptake of soluble but not of cell-associated antigen for cross-presentation. J. Immunol. 176, 6770-6776.
Dang Y, K. K., 2007. Tumor antigen-specific T cell expansion is greatly facilitated by in vivo priming. Clin. Cancer Res. 13, 6-12.
Fujiki, T., Tsuji, A., Matsumoto, S., Yamashita, M., Teruya, K., Shirahata, S., Katakura, Y., 2010. Generation of a human anti-tumor necrosis factor-α monoclonal antibody by in vitro immunization with a multiple antigen peptide. Biosci. Biotechnol. Biochem. 74, 1836-1840.
Geisberger, R., Königsberger, S. and Achatz, G., 2006. Membrane IgM influences membrane IgD mediated antigen internalization in the b cell line Bcl1. Immunol. Lett. 102, 169-176.
Germain, R. N. and Jenkins, M. K., 2004. In vivo antigen presentation. Curr. Opin. Immunol. 16, 120-125.
Köhler, G. and Milstein, C., 1975. Continuous cultures of fused cells secreting antibody of predefined specificity. Nature 256, 495-497.
Köhler, G. and Milstein, C., 1976. Derivation of specific antibody producing tissue culture and tumor cell lines by cell fusion. Eur. J. Immunol. 6, 511-519.
Levan-Petit, I., Lelievre, E., Barra, A., Limosin, A., Gombert, B., Preud'homme, J. L., Lecron J. C., 1999. Th2 cytokine dependence of IgD production by normal human B cells. Int. Immunol. 11, 1819-1828.
Liu Y. J., de Bouteiller O. and Fugier-Vivier I., 1997. Mechanisms of selection and differentiation in germinal centers. Curr. Opin. Immunol. 2, 256-262.
Lutz, M.B., Kukutsch, N., Ogilvie, A.L.J., Röβner, S., Koch, F., Romani, N., Schuler, G., 1999. An advanced culture method for generating large quantities of highly pure dendritic cells from mouse bone marrow. J. Immunol. Meth. 223, 77-92.
Magari, M., Kanehiro, Y., Todo, K., Ikeda, M., Kanayama, N., Ohmori, H., 2010. Enhancement of hypermutation frequency in the chicken B cell line DT40 for efficient diversification of the antibody repertoire. Biochem. Biophys. Res. Commun. 396, 353-358.
Mishell, R. I. and Dutton, R. W., 1966. Immunization of normal mouse spleen cell suspension in vitro. Science 153, 1004-1006.
Poltoratsky, V., Prasad, R., Horton, J. K., Wilson, S. H., 2007. Down-regulation of DNA polymerase beta accompanies somatic hypermutation in human BL2 cell lines. DNA Repair 6, 244-253.
Pulendran, B., Tang, H. and Manicassamy, S., 2010. Programming dendritic cells to induce TH2 and tolerogenic responses. Nature Immunology 11, 647-655.
Siddiqui, M. Z., 2010. Monoclonal antibodies as diagnostics; an appraisal. Indian J. Pharm. Sci. 72, 12-17.
Wang, W., Wang, E. Q. and Balthasar, J. P., 2008. Monoclonal antibody pharmacokinetics and pharmacodynamics. Clin. Pharmacol. Ther. 84, 548-558.

## Claims

1. In vitro method for production of antigen-specific antibodies, comprising
- provision of naïve dendritic cells in cell culture,
- activation of dendritic cells with target antigen,
- addition of naïve T cells and naïve B cells in co-culture with activated dendritic cells,
and
- activation of T cells and B cells leading to B cell production of said antibodies.

2. Method according to the preceding claim, **characterised in that**
the naive dendritic cells are generated by in vitro differentiation of precursor cells, such as stem cells of multipotent or pluripotent properties, preferably via granulocyte macrophage colony-stimulating factor (GM-CSF) treatment.

3. Method according to the preceding claim, **characterised in that**
the precursor cells are hematopoietic stem cells or monocytes, preferably obtained from blood, lymph nodes or bone marrow of a subject.

4. Method according to any one of the preceding claims, **characterised in that** the target antigen is the antigen against which the antibodies to be produced are directed, so that the antibodies preferably specifically bind the target antigen under appropriate conditions, whereby the target antigen can be any material against which an antibody is directed, such as a protein, peptide, nucleic acid, such as DNA or RNA, sugar molecule, virus and/or bacteria.

5. Method according to any one of the preceding claims, **characterised in that** the naïve T cells are naïve helper T lymphocytes, preferably isolated from blood, lymph nodes or spleen of non-immunized subjects.

6. Method according to any one of the preceding claims, **characterised in that** the naïve B cells are naïve B lymphocytes, preferably isolated from blood, lymph nodes or spleen of non-immunized subjects.

7. Method according to any one of the preceding claims, **characterised in that**
- the dendritic cells are activated via treatment with target antigen, preferably in combination with activation stimuli such as lipopolysaccharide (LPS) or others, to process and present target antigen or fragments thereof on the cell surface,
- the dendritic cells are treated with Th2 inducing stimuli, such as IL-12 siRNA, IL-12 blocking antibody or IL-4, to induce Th2 polarisation of naive T cells
- the T cells are activated via physical contact with the activated dendritic cells, preferably causing proliferation of T cells activated specifically for the target antigen or fragment thereof,
- the B cells are activated by activated T cells to produce antigen-specific antibodies directed against the target antigen or fragment thereof, and/or
- the T and B cells are provided in co-culture either separately, one after the other or simultaneously.

8. Method according to any one of the preceding claims, **characterised in that** the B cells, preferably those that produce target antigen-specific antibodies, are fused with myeloma cells to form hybridoma cells.

9. Method according to any one of the preceding claims, **characterised in that** the activated antigen-producing hybridoma cells are subsequently selected for mono-clonal cell culture, or that the desired antigen-producing B cells are selected before fusing with myeloma cells.

10. Method according to the preceding claim, **characterised in that**
- selection is carried out via a bridge antibody that binds both the hybridoma cell surface and the target antigen, whereby the hybridoma cells are treated with the bridge antibody and target antigen, so that the antigen-specific antibody produced by the hybridoma binds the antigen, and the antigen-specific antibody is detected via binding to a further antibody labelled with fluorescence or via other means; or
- selection is carried out via the target antigen (or desired fragment thereof) labelled with a fluorescent marker or other means, whereby the target antigen is administered to the hybridoma cells and binding between antigen-specific antibody and antigen is measured.

11. Method according to any one of the preceding claims, **characterised in that** the antigen-specific antibodies are monoclonal antibodies.

12. Method according to any one of the preceding claims, **characterised in that** the method comprises of the following steps, preferably in the following order:
- Collection of blood from subject;
- Isolation of hematopoietic stem cells or monocytes;
- Differentiation of hematopoietic stem cells into naïve dendritic cells in cell culture;
- Activation of dendritic cells with target antigen (antigen of choice);
- Addition of naïve T and B lymphocytes in co-culture;
- Incubation for between 2 and 6 days, preferably between 3 and 5 days, more preferably 4 days;
- Test for target antigen-specific antibody production;
- Fusion of B cells, preferably those that produce target antigen-specific antibodies, with myeloma cells to form hybridoma cells;
- Selection of hybridoma cells that produce target antigen-specific antibodies for monoclonal expansion.

13. Activated antigen-specific antibody producing B cell, antigen-specific antibody producing hybridoma cell, or cell line derived thereof, that is generated by the method of any one of the preceding claims.

14. Antibody produced according to the method of any one of the preceding claims for use in diagnostic or therapeutic applications.

15. Kit, comprising components required in order to carry out the method described herein, preferably means for labelling the cells, agents and/or detection antibodies, cell culture medium and/or reagents for driving differentiation of the cells and/or containers or means for cell culture and/or extraction.
